# EUROPÄISCHE PATENTSCHRIFT

(11) **EP 0 424 804 B1**
(45) Veröffentlichungstag und Bekanntmachung des Hinweises auf die Patenterteilung: **22.03.1995**
(21) Anmeldenummer: 90119989.3
(22) Anmeldetag: 18.10.1990
(51) Int. Cl.: C07C 53/10, C07C 51/43

(54) **Verfahren zur Herstellung von granuliertem Natriumacetat-Trihydrat**
Process for preparing granulated sodium acetate trihydrate
Procédé de préparation d'acétate de sodium granulé

(30) Priorität: 24.10.1989 DD 333833
(43) Veröffentlichungstag der Anmeldung: 02.05.1991
(73) Patentinhaber: GLATT INGENIEURTECHNIK GmbH, D-99423 Weimar (DE)
(72) Erfinder: Böber, Reinhard, Dipl.-Ing., O-5300 Weimar (DE); Peter, Siegfried, Dipl.-Ing., O-5300 Weimar (DE); Wand, Bernhard, Dipl.-Ing., O-5320 Apolda (DE); Freudenberg, Werner, Dr.-Ing., O-8027 Dresden (DE); Kniest, Steffen, Dipl.-Ing., O-8036 Dresden (DE); Wegner, Joachim, Dipl.-Ing., O-8210 Freital (DE); Heidrich, Matthias, Dr.-Ing., O-8021 Dresden (DE); Hönisch, Dietrich, O-8020 Dresden (DE)
(74) Vertreter: Beetz & Partner Patentanwälte

(56) Entgegenhaltungen:
- EP-A- 0 141 437
- DD-A- 145 855
- PATENT ABSTRACTS OF JAPAN, Band 4, Nr. 34 (C-3)[516], 22. März 1980;& JP-A-557 236 (TAKEDA YAKUHIN KOGYO) 19-01-1980

## Beschreibung

Die Erfindung betrifft ein Verfahren zur Herstellung von granuliertem Natriumacetat-Trihydrat aus dessen wäßriger Lösung.

Die Herstellung von Natriumacetat-Trihydrat aus dessen wäßriger, ungesättigter/gesättigter Lösung erfolgt üblicherweise durch Eindampfen oder durch Kristallisation mit anschließender Fest-Flüssig-Trennung und Nachtrocknung oder im Falle einer gesättigten Lösung durch Kristallisation mit anschließender Vermahlung. Kennzeichnend für diese diskontinuierlichen Verfahren ist ein hoher apparatetechnischer und energetischer Aufwand. Dieser läßt sich durch das aus der DD-A-145 855 bekannte kontinuierliche Verfahren der Sprühkristallisation reduzieren. Hierbei wird im Sprühturm die heiße Natriumacetatlösung mittels Druckdüse oder Zerstäuberscheibe fein in einem kalten Luftstrom verteilt, und man erhält ein fließfähiges, aber sehr feinkörniges und stark staubendes Natriumacetat-Trihydrat. Zur Kühlung sind je kg Salz erhebliche Mengen Luft erforderlich. Es werden dabei große Apparate benötigt. Wegen seines hygroskopischen Verhaltens läßt sich dieses Natriumacetat-Trihydrat schlecht oder nur mit großem Aufwand lagern und transportieren.

EP-A-0 141 437 offenbart die Herstellung von Körnern durch Wachstum von Keimen in einer Wirbelschicht, indem man ein Flüssigmaterial daran erstarren läßt. Dieses wird in der Schicht aufwärts mit Hilfe einer Spritzeinrichtung gespritzt, die mit einem Zentralkanal, durch den das Flüssigmaterial zugeführt wird, und einem dazu konzentrischen Kanal versehen ist, der einen starken Gasstrom fördert, wobei das Flüssigmaterial den Gasstrom kontaktiert und mit dem Gasstrom zu einer Verdünnungszone gefördert wird, wo das Wachstum der Keime stattfindet. Granuliertes Natriumacetat-Trihydrat erwähnt dieses Dokument nicht.

Der Erfindung liegt die Aufgabe zugrunde, ein Verfahren zu entwickeln, mit dem durch Granulation und Kristallisation ein gleichmäßiges, gut lager-, transport- und dosierfähiges, abriebfestes, staubarmes sowie gut lösliches und klumpfreies Natriumacetat-Trihydrat-Granulat mit engem Kornspektrum des Endproduktes gewonnen wird.

Diese Aufgabe wird erfindungsgemäß durch das Verfahren gemäß Patentanspruch 1 gelöst.

Vorteilhafte Ausgestaltungen der Erfindung sind in den Unteransprüchen gekennzeichnet.

Erfindungsgemäß wird eine gleichzeitige Granulation und Kristallisation dadurch erreicht, daß die heiße, wäßrige, hoch konzentrierte Natriumacetatlösung in eine Wirbelschicht mit klassierendem Abzug eingedüst wird. Die Lösung benetzt dabei die Oberfläche der wirbelnden Partikel und kristallisiert auf dieser. Die infolge Abkühlung und Kristallisation frei werdende Wärme wird einerseits durch die Verdunstung des Lösungsmittels verbraucht, andererseits an das Kühlmedium abgegeben.

Der zurückbleibende Feststoff läßt die Granalien anwachsen, bis diese die Wirbelschicht durch ein zentrales Abzugsrohr verlassen. Beeinflußt werden der Austrag und die Granulatgröße durch entsprechende Wahl der Austragsluftgeschwindigkeit im Abzugsrohr.

Es wird ein enges Kornspektrum des Endproduktes im Korngrößenbereich von 0,5 bis 10 mm, vorzugsweise von 1,5 bis 5 mm, erreicht.

Das so erhaltene Natriumacetat-Trihydrat ist gleichmäßig, abriebfest, staubarm, wenig hygroskopisch, gut förder-, lager- und dosierfähig und gut löslich.

In einem anschließenden Kühler kann gegebenenfalls eine weitere Kühlung der erzeugten Granalien erfolgen. Die in der Wirbelschicht zu Beginn und zur Aufrechterhaltung des Granulierprozesses zuzuführenden Keime können aus dem Fertigprodukt durch Mahlung hergestellt werden. Der vom Entstauber abgeschiedene Staub kann separat gebunkert oder auch in die Wirbelschicht, wobei er als Keimmaterial wirkt, zurückgeführt werden.
Zur Kühlung wird Luft, vorzugsweise Umgebungsluft, des Temperaturbereiches von -40 bis +35 °C benutzt, die im Verhältnis von 6 bis 40, vorzugsweise 7 bis 10 m³ Luft/kg Lösung eingesetzt wird. Die Ablufttemperatur beträgt max. 42 °C, die Fertigprodukttemperatur 30 bis 40 °C. Der Einsatz konditionierter Luft bewirkt weitgehend konstante Prozeßverhältnisse.
Die Konzentration der mittels Dralldruckdüse oder Injektionsdüse mit vorgewärmter Luft eingebrachten Natriumacetatlösung beträgt 50 bis 60 %, vorzugsweise 55 bis 58 %. Gegenüber der Sprühkristallisation läßt sich das Luft/Lösung-Verhältnis u.a. aufgrund der größeren Menge verdunsteten Wassers pro kg Lösung in der Wirbelschicht deutlich verringern. Zusätzliche Bindemittel können verwendet werden, sind aber nicht erforderlich. Möglich ist auch eine chargenweise Granulierkristallisation, wobei die Korngröße durch die Prozeßdauer bestimmt wird.

Die Erfindung wird nachstehend an einem Ausführungsbeispiel näher erläutert.
In einem Wirbelschichtreaktor wird durch Kühlungsluft mit einer Temperatur von 23 °C oberhalb des Anstrombodens eine Wirbelschicht mit arteigenem Material erzeugt. In diese Wirbelschicht wird 90 °C heiße Natriumacetatlösung mit einem Feststoffgehalt von 56 % mittels Injektionsdüse und 90 °C heißer Luft als Treibmittel gesprüht. Beim Kristallisieren bildet das Natriumacetat mit dem Lösungsmittel Wasser ein Trihydrat, das agglomeriert. Das überschüssige Wasser (7,5 g/100 g Lösung) nimmt die bei der Kristallisation frei werdende Wärme auf, verdunstet und wird mit der sich auf 40 °C erwärmenden Kühlungsluft ausgetragen. Es ist ein Durchsatzverhältnis von 8,5 m³ Luft pro kg Lösung erforderlich.
Das Granulat verläßt die Wirbelschicht mit einer Temperatur von 36 °c und einem durchschnittlichem Kristallwassergehalt von 39,5 % bei einer vorbestimmten Korngröße von ca. 2 mm durch ein klassierendes Abzugsrohr. Die Trocknerabluft wird mittels eines Zyklons und Naßwäschers entstaubt. Der Wirbelschicht werden ca. 8 % Keime, bezogen auf die Menge ausgetragenen Fertigprodukts, zugeführt, das aus Fertiggranulat in einem geeigneten Mahlaggregat hergestellt wird. Die Schüttdichte des gleichmäßigen, abriebfesten, staubarmen, gut förder-, dosier- und lagerfähigen sowie gut löslichen Granulats beträgt ca. 800 kg/m³.

Das mit dem erfinderischen Verfahren hergestellte Erzeugnis stellt ein Zwischenprodukt in der chemischen Industrie zur Herstellung einer Vielzahl von Produkten dar. Es ist z.B. als Stabilisator für die Gummiherstellung oder als Konservierungsmittel in der Lebensmittelindustrie verwenbar.

## Patentansprüche

1. Verfahren zur Herstellung von granuliertem Natriumacetat-Trihydrat aus einer hoch konzentrierten, heißen wäßrigen Natriumacetatlösung,
**dadurch gekennzeichnet,**
daß die heiße Lösung mit einer Konzentration von 50 - 60 % mittels Dralldruck- oder Injektionsdüse in eine Wirbelschicht eingedüst wird, daß dabei die Granulation, Kristallisation und Verdunstung in einem Verfahrensschritt in konditionierter und/oder mit denen der Umgebung entsprechenden Parametern versehener Luft des Temperaturbereiches von -40 bis +35 °C, im Verhältnis von 6 bis 40 m³ Luft/kg Lösung, erfolgen, und daß ohne oder mit Zusatz von Bindemitteln oder anderen Granulierhilfsmitteln ein Natriumacetat-Trihydrat mit einer Temperatur unter 40 °C und einer vorbestimmten Korngröße im Bereich von 0,5 bis 10 mm gebildet und über einen klassierenden Luftstrom ausgetragen wird.

2. Verfahren nach Anspruch 1,
dadurch gekennzeichnet,
daß die Granulation, Kristallisation und Verdunstung diskontinuierlich im Chargenbetrieb erfolgen.

3. Verfahren nach Anspruch 1 oder 2,
dadurch gekennzeichnet,
daß die heiße Lösung mit einer Konzentration von 55 - 58 % eingedüst wird.

4. Verfahren nach Anspruch 1 oder 2,
dadurch gekennzeichnet,
daß das Luft/Lösungs-Verhältnis 7 bis 10 m³ Luft/kg Lösung beträgt.

5. Verfahren nach Anspruch 1 oder 2,
dadurch gekennzeichnet,
daß das Natriumacetat-Trihydrat mit einer Korngröße im Bereich von 1,5 bis 5 mm gebildet wird.

## Claims

1. A process for the production of granulated sodium acetate trihydrate from a highly concentrated hot aqueous sodium acetate solution, characterised in that the hot solution is injected into a fluidised bed in a concentration of 50 to 60% by means of a spinning pressure or injection nozzle, in that the granulation, crystallisation and evaporation are effected in a process step in conditioned air and/or air having parameters corresponding to those of the environment, said air being in the temperature range from -40 to +35°C, in a ratio of 6 to 40 m³ air per kg solution, and in that with or without the addition of binders or other granulation adjuvants a sodium acetate trihydrate is formed at a temperature below 40°C and with a predetermined grain size in the range from 0.5 to 10 mm and is discharged via a classifying airstream.

2. A process according to claim 1, characterised in that the granulation, crystallisation and evaporation are effected discontinuously in batch operation.

3. A process according to claim 1 or 2, characterised in that the hot solution is injected in a concentration of 55 - 58%.

4. A process according to claim 1 or 2, characterised in that the air/solution ratio is 7 to 10 m³ air per kg of solution.

5. A process according to claim 1 or 2, characterised in that the sodium acetate trihydrate is formed with a grain size in the range from 1.5 to 5 mm.

## Revendications

1. Procédé pour la préparation de trihydrate d'acétate de sodium granulé à partir d'une solution d'acétate de sodium hautement concentrée, chaude, aqueuse, caractérisé en ce qu'on injecte la solution chaude avec une concentration de 50-60 % à l'aide d'une buse sous pression à torsion ou d'injection dans un lit fluidisé, en ce que ce faisant la granulation, la cristallisation et l'évaporation ont lieu dans une étape du procédé dans de l'air conditionné et/ou muni des paramètres correspondants de l'air ambiant de celui-ci du domaine de température de -40 à +35°C, selon un rapport de 6 à 40 m³ d'air/kg de solution et en ce qu'un trihydrate d'acétate de sodium avec une température inférieure à 40°C et une grosseur de grain prédéterminée dans un domaine de 0,5 à 10 mm est formé sans ou avec l'ajout d'agents liants ou d'autres agents auxiliaires de granulation, et est évacué par un courant d'air classifiant.

2. Procédé selon la revendication 1, caractérisé en ce que la granulation, la cristallisation et l'évaporation s'effectuent en discontinu dans un fonctionnement discontinu.

3. Procédé selon la revendication 1 ou 2, caractérisé en ce qu'on injecte la solution chaude avec une concentration de 55-58 %.

4. Procédé selon la revendication 1 ou 2, caractérisé en ce que le rapport air solution est de 7 à 10 m³ d'air/kg de solution.

5. Procédé selon la revendication 1 ou 2, caractérisé en de que le trihydrate d'acétate de sodium est formé avec une grosseur de grain dans un domaine de 1,5 à 5 mm.
